(19) Europäisches Patentamt
European Patent Office
Office européen des brevets

(11) **EP 3 767 263 B1**

(12) **EUROPÄISCHE PATENTSCHRIFT**

(45) Veröffentlichungstag und Bekanntmachung des Hinweises auf die Patenterteilung:
**21.12.2022  Patentblatt 2022/51**

(21) Anmeldenummer: **19186604.5**

(22) Anmeldetag: **16.07.2019**

(51) Internationale Patentklassifikation (IPC):
**G01N 33/205** (2019.01)   **G01N 23/2202** (2018.01)
**G01K 15/00** (2006.01)   **G01K 1/14** (2021.01)
**G01J 5/00** (2022.01)
**G01N 1/38** (2006.01)

(52) Gemeinsame Patentklassifikation (CPC):
**G01K 1/146; G01K 15/005; G01N 23/2202;**
**G01N 33/205;** G01J 5/004; G01N 1/44;
G01N 2001/386

(54) **AUFSCHLUSSVORRICHTUNG MIT EINER SCHMELZTEMPERATURMESSVORRICHTUNG UND KALIBRIERVERFAHREN**

FUSION DEVICE COMPRISING A MELT TEMPERATURE MEASURING DEVICE AND CALIBRATION METHOD

DISPOSITIF DE DISSOLUTION COMPRENANT UN DISPOSITIF DE MESURE DE LA TEMPÉRATURE DE FUSION ET PROCÉDÉ DE CALIBRATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**20.01.2021  Patentblatt 2021/03**

(73) Patentinhaber: **Herzog Maschinenfabrik GmbH & Co. KG**
**49086 Osnabrück (DE)**

(72) Erfinder:
• **Herzog, Jan**
**49124 Georgsmarienhütte (DE)**
• **Mehling, Andre**
**49082 Osnabrück (DE)**
• **Lischka, Martin**
**49074 Osnabrück (DE)**

(74) Vertreter: **Stolmár & Partner**
**Patentanwälte PartG mbB**
**Blumenstraße 17**
**80331 München (DE)**

(56) Entgegenhaltungen:
**EP-A1- 1 236 982     CA-C- 1 280 189**
**DE-A1- 10 346 993     FR-A1- 2 523 302**
**LU-A1- 88 680     US-A1- 2019 093 951**

**Beschreibung**

Gebiet der Erfindung

Die Erfindung betrifft eine Aufschlussvorrichtung mit einer Temperaturmessvorrichtung zum Kalibrieren der Schmelztemperaturerfassung, und ein entsprechendes Kalibrierverfahren.

Stand der Technik

Das sogenannte Borat- Aufschlussverfahren ist ein wichtiger Bestandteil der Materialanalyse durch Verfahren der Röntgenfluoreszenzanalyse (RFA), Massenspektrometrie mit induktiv gekoppeltem Plasma oder Atomabsorption (AA). Für die RFA wird eine Probe beispielsweise mit einem Überschuss an Lithiumborat aufgeschlossen und in Form einer Glasperle mit einer flachen Oberfläche ausgegossen. Während des Aufschlussprozesses werden die Materialphasen der Probe in glasähnliche Borate umgewandelt, was zu einer homogenen Aufschlussperle führt. Zunächst wird das fein gemahlene Probenmaterial mit einem Borat- Aufschlussmittel in einem Tiegel gemischt, welcher zu 95 % aus Platin und 5 % aus Gold besteht. Dann wird der Tiegel auf Temperaturen über 1000 °C erhitzt, bis die Probe in dem geschmolzenen Aufschlussmittel gelöst ist.

Mit Hilfe des Aufschlusses können gegenüber anderen Verfahren wie z.B.

Tablettenpressung analytische Fehler infolge inhomogener Partikelverteilung, mineralogischer Effekte und unzureichender Oberflächenqualität reduziert werden. Dieser positive Effekt lässt sich durch folgende Faktoren erklären: Erstens können sich Proben, die zwar eine identische chemische Zusammensetzung haben, durch ihre Mineralogie und Partikelgröße voneinander unterscheiden. Dies kann zu unterschiedlichen Zählraten in dem Analysegerät führen. Der Aufschlussprozess eliminiert diese Faktoren und erhöht dadurch die Messgenauigkeit. Zweitens findet beim Aufschluss durch das Hinzufügen von Aufschlussmittel eine Verdünnung statt. Dies führt sowohl zu einer Verringerung der Interaktion zwischen den zu analysierenden Elementen als auch zu einer Reduzierung des sogenannten Matrix- Effektes. Drittens erleichtert der Aufschluss die Durchführung einer Kalibrierung, da dieses Aufschlussverfahren u.a. die Verwendung synthetischer Standards ermöglicht.

Es stehen unterschiedliche Aufschlusssysteme mit unterschiedlichen Öfen zur Erhitzung des Borat- Probengemisches innerhalb des Platin-/ Gold- Tiegels zur Verfügung: Gasöfen, elektrische Widerstandsöfen und induktive Öfen. Gasöfen sind preisgünstig, jedoch restriktiven Sicherheitseinschränkungen unterworfen. Darüber hinaus ist eine Temperaturmessung in Gasöfen nur indirekt in der Nähe des Tiegels möglich und wird nur selten durchgeführt, da die Messung nicht die Temperatur des Aufschlusses zeigt sondern nur als Indikator gilt.Die Zieltemperatur wird daher in der Regel lediglich indirekt über den Gasstrom gesteuert. Entsprechend ist die erreichbare Temperaturgenauigkeit in solchen Geräten auch nur relativ gering. Elektrische Widerstandsöfen ermöglichen durch die Verwendung von Thermofühlern in der Ofenkammer eine präzise Temperatursteuerung. Jedoch besitzen diese Öfen träge Heizsysteme, so dass die Verwirklichung eines Temperaturanstiegs und -abfalls in der Heizkammer zeitaufwändig ist. Ein weiterer Nachteil ist, dass die Durchmischung der Schmelze im Tiegel nicht besonders suffizient ist, da der Tiegel lediglich um eine Achse gedreht werden kann.

Bei induktiven Systemen wird Hochfrequenz- Elektrizität verwendet, um elektrisch leitfähige Materialien wie z. B. den Gold-/ Platin- Tiegel aufzuheizen. Dabei wird ein hochfrequent alternierender Strom durch eine Spule geleitet. Dadurch wird ein starkes, rasch wechselndes Magnetfeld in dem von der Spule umgebenen Raum induziert. Spule und Tiegel bilden zusammen eine Art Transformator. Die Spule stellt die Primärwicklung dar, in die die elektrische Energie eingegeben wird. Der Tiegel fungiert als Sekundärwicklung mit einer Windung, welche kurzgeschlossen wird. Dies bewirkt, dass ein hoher Strom durch den Tiegel fließt (sog. Wirbelstrom oder "eddy current"). Darüber hinaus spielt ein sogenannter "skin effect" eine Rolle, welcher den alternierenden Strom dazu zwingt, in einer dünnen Schicht Richtung Tiegeloberfläche zu fließen. Der "skin effect" führt zu einem deutlichen Anstieg des Tiegelwiderstandes gegenüber dem Wirbelstrom und erhöht dadurch zusätzlich den Aufheizeffekt. Die in dem Tiegel befindliche Mischung aus Probenmaterial und Flussmittel wird also bei Induktionsverfahren nicht direkt, sondern indirekt über die Erwärmung des Tiegels erhitzt und geschmolzen.

Mit Hilfe des Induktionsverfahrens kann sehr schnell die gewünschte Temperatur erreicht und zwischen unterschiedlichen Temperaturstufen gewechselt werden. Dies erweist sich beispielsweise als vorteilhaft, wenn Proben deutlich unterhalb des Schmelzpunktes oxidiert werden müssen. Nach Abschluss der Oxidation, bei beispielsweise 400 °C, kann dann innerhalb weniger Sekunden auf die Schmelztemperatur von beispielsweise 1100 °C gewechselt werden. Bei Widerstandsöfen würde dieser Schritt in der Regel mindestens 15 bis 30 Minuten dauern.

Außerdem erlaubt die Induktionsmethode, die Tiegeltemperatur direkt und kontinuierlich mit Hilfe eines Infrarot- Pyrometers zu messen. Dabei fängt das optische System eines Infrarotthermometers die von einem kreisförmigen Messfleck auf dem Tiegel abgestrahlte infrarote Energie auf und fokussiert sie auf einen Detektor. Dieser wandelt die empfangene Infrarotstrahlung in elektrische Signale um, die von der nachfolgenden Elektronik als Temperaturwerte ausgegeben werden. Die gemessene Pyrometertemperatur kann als Stellgröße in der Temperatursteuerung verwendet werden, wodurch die Zieltemperatur auf sehr schnelle Art und Weise erreicht und in einem engen Bereich konstant gehalten werden kann. Damit durch das Pyrometer eine präzise Temperaturmessung vorgenommen werden kann, muss der sogenannte Emissionsgrad $\varepsilon$ des zu messenden Objektes eingestellt werden. So entspricht der Emissionsgrad eines idealen schwarzen Körpers dem Wert 1. Bei Objekten mit glänzenden Oberflächen wie z.B. Metalle, so genannte graue Körper, liegt der Emissionsgrad unterhalb von 1. Allerdings unterliegt die Messung der Schmelztemperatur, insbesondere bei Tiegelmetallen wie Platin, einer zunehmenden Ungenauigkeit mit steigender Temperatur, die sich für jeden Tiegel unterscheidet. Somit ist die Messung mittels Pyrometer bei einem Induktionsverfahren über die gesamte Lebenszeit und Temperaturbreite relativ ungenau.

EP1236982A1 offenbart die Kalibrierung eines Strahlungsthermometers mittels einer Fixpunktzelle in einem Ofen mit bekannter Innentemperatur.

Darstellung der Erfindung

Aufgabe der vorliegenden Erfindung ist es, eine Induktionsaufschlussvorrichtung und ein entsprechendes Verfahren bereitzustellen, bei der die Genauigkeit der Schmelztemperaturmessung erheblich verbessert ist. Diese Aufgabe wird gelöst durch eine Aufschlussvorrichtung nach Anspruch 1 und durch ein Verfahren nach Anspruch 8. Weitere, die Erfindung ausgestaltende

Merkmale sind in den jeweiligen abhängigen Ansprüchen enthalten.

Eine erfindungsgemäße Aufschlussvorrichtung zum Kalibrieren der Schmelztemperaturerfassung, umfasst eine Temperaturmessvorrichtung mit einem bewegbaren und zumindest teilweise stangenförmigen Temperaturfühler, der in einen Tiegel einsteckbar ist, eine Bewegungseinrichtung zum Bewegen des Temperaturfühlers zwischen einer Initialposition und einer Messposition und eine Lagerplatte, auf der der stangenförmige Temperaturfühler und die Bewegungseinrichtung gelagert sind und die in einer Aufschlussvorrichtung montierbar ist. Mit dieser Temperaturmessvorrichtung ist eine einfache, sichere und schnelle Kalibrierung einer Schmelztemperaturerfassung in der Aufschlussvorrichtung möglich. Da die Genauigkeit der Temperaturmessung eines (Platin/Gold-)Tiegels mit Hilfe der Strahlungserfassungsvorrichtung ( Pyrometer) in einem Induktions-Aufschlusssystem maßgeblich durch zwei Faktoren beeinträchtigt wird, nämlich der temperaturabhängigen Änderung des Emissionsgrades und der alterungsbedingten Änderung des Emissionsgrades, kann durch eine solche Messvorrichtung der entsprechende Zusammenhang zwischen der Hitzestrahlung und der tatsächlichen Schmelztemperatur in kurzer Zeit zuverlässig bestimmt werden und danach eine äußerst genaue Temperaturmessung der Schmelztemperatur durch die Strahlungserfassungsvorrichtung durchgeführt werden. Denn das Problem, dass mit steigender Temperatur des Tiegels sich auch die Strahlung des Tiegels ändert und dadurch eine zunehmende Ungenauigkeit der Pyrometermessung bei sich ändernden Temperaturen bewirkt wird, so dass es zu einem zunehmenden Unterschied zwischen der Pyrometer- Temperatur und der tatsächlichen Temperatur in der Schmelze kommt, wird durch die mögliche Kalibrierung aufgrund der Temperaturmessvorrichtung dauerhaft vermieden. Ebenso werden die alterungsbedingten Änderungen des Emissionsgrads beim Strahlungsmessungsabgleich inhärent berücksichtigt. Denn auch mit zunehmender Anzahl der durchgeführten Aufschlüsse verändert sich das Mikrogefüge innerhalb des Tiegels. Dies führt dazu, dass ein initial matter Tiegel zunehmend glänzend und ein initial glänzender Tiegel zunehmend matter wird. Diese Veränderung der Reflexionseigenschaften an der Außenseite des Tiegels führt gleichfalls zu einer langsamen Änderung des Emissionsgrades des Tiegels im Laufe seiner Lebensdauer. Die Emissionsgradänderung ist je nach Tiegeltyp während der ersten ungefähr 200 - 500 Schmelzaufschlüsse deutlich ausgeprägt und wird nachfolgend immer geringer.

Der Temperaturfühler der Temperaturmessvorrichtung ist vorzugsweise mittels zweier Backen fixiert und insbesondere in einer Nut der Backen angeordnet. Dies lässt eine flexible Ausrichtung zu, bei der die endgültige Position aber sicher festgelegt ist.

Der Temperaturfühler ist bevorzugt gebogen ausgebildet. Dadurch kann er platzsparend in der Aufschlussvorrichtung angeordnet und bewegt werden.

Eine Vorrichtung umfasst bevorzugt ferner einen Hitzeschild, der eine insbesondere schlitzförmige Aussparung aufweist, durch die der Temperaturfühler hindurch bewegbar ist. Der Temperaturfühler ist vorzugsweise rotatorisch gelagert und kann insbesondere durch einen elektrischen, pneumatischen oder hydraulischen Antrieb automatisiert sein. Alternativ kann der Temperaturfühler auch durch einen Handhebel bewegbar sein, der bspw. als längliche Stange mit optionalem Handgriff ausgebildet sein kann. Durch die rotatorische Aufhängung des Temperaturfühlers ist die Bewegung von der Startposition zur Messposition platzsparend auszuführen.

Die Vorrichtung weist ferner vorzugsweise zumindest einen Anschlag zum Begrenzen der Bewegung der Bewegungseinrichtung auf. Dadurch kann auf eine einfache Weise die vordefinierte Messposition aber auch eine Initialposition bestimmt und zuverlässig erreicht und eingehalten werden.

Eine erfindungsgemäße Aufschlussvorrichtung mit einer Temperaturmessvorrichtung umfasst ferner eine Hitzestrahlungserfassungseinrichtung,

einen Tiegelstellplatz, der in Bezug auf die Messvorrichtung vordefiniert ist, eine Induktionseinheit, mit der der Tiegelinhalt aufheizbar ist, und eine Steuereinheit, die den Abgleich zwischen der durch die Temperaturmessvorrichtung gemessenen Schmelztemperatur und der durch die Hitzestrahlungserfassungseinrichtung erfassten Strahlung durchführt. Eine solche Aufschlussvorrichtung kann unkompliziert regelmäßige Erst- und Rekalibrierungen durchführen.

Ein erfindungsgemäßes Verfahren zum Kalibrieren einer Temperaturmesseinrichtung einer Aufschlussvorrichtung, umfasst die Schritte Bereitstellen eines Schmelztiegels, induktives Aufheizen eines Schmelztiegels mit Inhalt, so dass der Inhalt sich erwärmt und schmilzt, Fahren eines Temperaturfühlers an eine Messposition in der Schmelze und exaktes Messen der Inhaltstemperatur mit dem Temperaturfühler und Messen der Infrarotstrahlung des Tiegels, insbesondere eines vorbestimmten Messpunktes des Tiegels, mit einem Pyrometer, so dass die Strahlung (bspw. der Emissionsgrad $\varepsilon$) erfasst werden kann und in Beziehung zur Schmelztemperatur gesetzt wird. Insbesondere werden die Schritte des exakten Messens der Inhaltstemperatur mit dem Temperaturfühler und das Erfassen der Strahlung (bspw. des Emissionsgrads $\varepsilon$) bei unterschiedlichen Temperaturen wiederholt und/oder zuerst mit einem unbenutzten Schmelztiegel durchgeführt und es dann in einem bestimmten Zeitabstand wiederholt wird, so dass die alterungsbedingte Änderung des Strahlungsverhaltens (bspw. des Emissionsgrads $\varepsilon$) erfasst wird. Durch dieses Kalibrierverfahren kann die Schmelztemperatur der Schmelze eines Tiegels in einer Aufschlussvorrichtung auf einfache und unkomplizierte Art zuverlässig bestimmt werden.

Kurze Beschreibung der Figuren

Figuren 1a und 1b zeigen eine isometrische Darstellung einer erfindungsgemäßen Temperaturmessvorrichtung in Start- und Messstellung;
Figur 2 zeigt eine Seitenansicht der Temperaturmessvorrichtung aus Figur 1;
Figuren 3a und 3b zeigen Ausführungen von Klemmbacken, mit denen der Temperaturfühler fixierbar ist; und
Figuren 4a und 4b zeigen zwei Vergleichsdiagramme ohne und mit Kalibrierung.

Beschreibung der bevorzugten Ausführungsformen

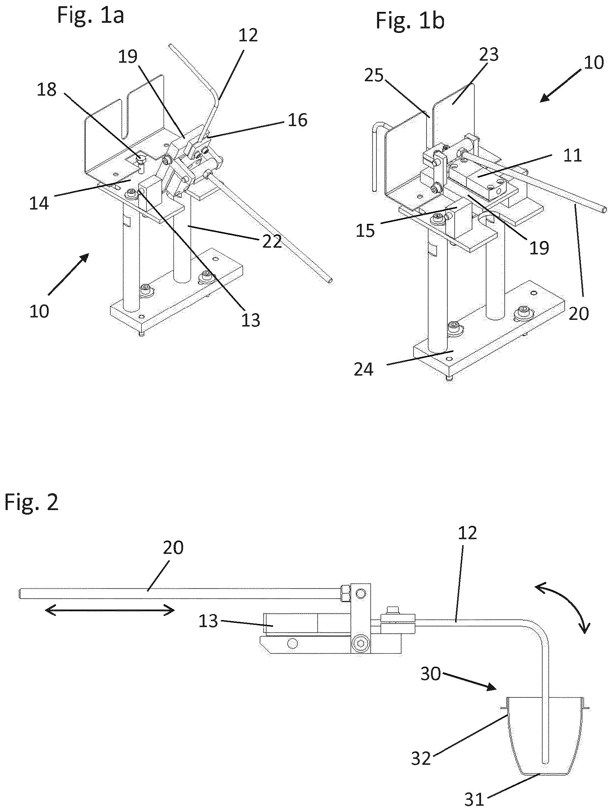

In den Figuren 1a und 1b ist eine Temperaturmessvorrichtung 10 für das Kalibrieren der Schmelztemperaturerfassung in einer Aufschlussvorrichtung in einer isometrischen Ansicht dargestellt. Figur 1a zeigt dabei die Stellung in einer Initialposition, Figur 1b die Stellung in einer Messposition (ohne Tiegel). Die Temperaturmessvorrichtung 10 ist für die Verwendung in einer Aufschlussvorrichtung mit einem Induktionsofen vorgesehen, bei dem die Schmelztemperatur der Schmelze in einem Tiegel 30 indirekt über die Infrarotstrahlung des Tiegels gemessen werden soll. Die Temperaturmessvorrichtung 10 weist einen Temperaturfühler 12 auf, mit dem die tatsächliche Schmelztemperatur der Schmelze in einem Tiegel 30 direkt gemessen werden soll. Der Temperaturfühler 12 ist auf einer Lagerplatte 14 zwischen einer Initialposition und einer Messposition bewegbar angeordnet. Die Messposition muss in Bezug auf den Tiegel 30 immer an der gleichen Stelle vorgesehen sein. Der Temperaturfühler ist daher idealerweise so auf der Lagerplatte 14 fixiert, so dass er nach der Montage lediglich zwischen der Initial- und der Messposition bewegbar ist, sich aber nicht mehr anderweitig bewegen kann, bspw. verdrehen. Dazu sind vorzugsweise Klemmbacken 16 vorgesehen, zwischen denen der Temperaturfühler 12 eingeklemmt werden kann. In einer oder beiden Backen 16 können dafür Lagernuten 17 ausgebildet sein, in der der Temperaturfühler 12 eingelegt sein kann um die die korrekte Ausrichtung sicherzustellen. Der Temperaturfühler 12 kann kabellos oder auch mit Kabeln mit einem Steuergerät verbunden sein, das die Daten des Temperaturfühlers 12 verarbeiten kann. Üblicherweise ist dies das Steuergerät der Aufschlussvorrichtung.

Der Temperaturfühler 12 ist vorzugsweise gebogen, insbesondere um 90°. Dadurch kann die Temperaturmessvorrichtung 10 auch bei den etwas engeren Platzverhältnissen in einer Aufschlussvorrichtung problemlos bewegt werden. Insbesondere die gebogene Ausgestaltung des Temperaturfühlers 12 begünstigt dessen rotatorische Lagerung, wie in den Figuren 1a, 1b und 2 dargestellt. Dazu ist der Temperaturfühler 12 auf einer Zwischenlagerung 19 befestigt, die hier durch die Klemmbacken 16 ausgebildet ist. Zwei Lagerelemente 15 und eine Achse 13 befestigen dann die Klemmbacken 16 an der Lagerplatte 14, so dass sich die Klemmbacken 16 relativ zur Lagerplatte 14 drehen können und den Temperaturfühler 12 so von der Initialposition in die Messposition bewegen. Die Bewegung des Temperaturfühlers 12 kann manuell durchführbar sein und bspw. durch eine Betätigungsstange 20 verwirklicht werden. Allerdings kann sie auch automatisiert sein. Dazu kommen unter anderem pneumatische, hydraulische oder elektrische Antriebe (Elektromotor) in Frage. Um die Rotationsfreiheit zu begrenzen kann ein Anschlag 18 vorgesehen sein, auf dem die Zwischenlagerung 19 in der Messposition des Temperaturfühlers 12 aufliegt und am weiteren herumschwenken gehindert wird. Der Anschlag kann aber auch an einer anderen Stelle oder auf andere Weise ausgeführt werden, bspw. als Drehbegrenzer der Achse 13. Ebenso kann die Bewegungsfreiheit des Temperaturfühlers 12 in der Initialposition durch einen Anschlag begrenzt werden.

Die Lagerplatte ist hier auf Beinen 22 angeordnet, um in der Aufschlussvorrichtung in einer Position auf Höhe des Tiegelstandorts angeordnet zu sein. Am Fuß der Beine 22 ist eine Standplattform 24 vorgesehen, mit der die Messvorrichtung 10 in der Aufschlussvorrichtung befestigt wird. Falls die Messvorrichtung 10 wieder demontiert werden soll, kann am Boden der Aufschlussvorrichtung ein Anschlag befestigt werden, so dass die exakte Position der Messvorrichtung 10 bei einer Neumontage wieder eingestellt werden kann.

Sowohl in der Lagerplatte 14 als auch in der Standplattform 24 können Langlöcher vorgesehen sein, um die Messvorrichtung in x- und y-Richtung auszurichten. In der Standplattform 24 können ferner Stiftlochbohrungen vorgesehen, um nach der Ausrichtung die gesamte Konstruktion auf dem Boden des Aufschlussgerätes definiert zu platzieren. Wenn die Kalibriervorrichtung nicht verwendet und demontiert wird, kann diese anhand der Stiftlochbohrungen in die exakt gleiche Position, wie vor der Demontage montiert werden, ohne diese wieder neu ausrichten zu müssen.

Ferner kann die Messvorrichtung auch einen Schild 23 aufweisen, der insbesondere zum Schutz des Steckers 11 des Temperaturfühlers 12 vor der Hitze vorgesehen ist. Der Schild 23 kann eine Aussparung 25 aufweisen, durch den der Temperaturfühler 12 hindurchbewegt werden kann den in den Tiegel 30 gelangt. Hier ist die Aussparung 25 als Schlitz ausgeführt und der Temperaturfühler 12 wird bei der Drehung durch den Schlitz 25 in den Tiegel 30 gedreht (Siehe Figuren 1a und 1b).

Wenn eine Aufschlussvorrichtung eine solche Messvorrichtung 10 umfasst, werden einige Elemente der Aufschlussvorrichtung für die Kalibrierung verwendet. Insbesondere der Tiegelstellplatz, auf dem der Tiegel 30 für den Aufschlussvorgang abgestellt ist, die Induktionseinheit, mit der der Tiegelinhalt erhitzt wird, die Strahlungsaufnahmevorrichtung wie einem Pyrometer, mit dem die Strahlung des Tiegels mit erhitztem Inhalt erfasst wird, und die Steuerungseinheit, um die erfasste Strahlung dann mit der von der Messvorrichtung 10 gemessenen Schmelztemperatur in Beziehung zu setzen.

Mittels dieser Messvorrichtung kann dann sowohl die temperaturabhängige als auch die alterungsbedingte Emissionsgradänderung kompensiert werden, indem die gemessene Pyrometertemperatur auf die tatsächliche Temperatur in der Schmelze zurückschließen lässt. Für einen Kalibriervorgang wird mit der Messvorrichtung die Temperatur in der Schmelze gemessen. Die wichtigsten Funktionen für die Messung der tatsächlichen Schmelztemperatur und der Kalibrierung sind den Temperaturfühler 12 ausrichten, den Temperaturfühler 12 fixieren, den Temperaturfühler 12 zum Tiegel 30 und in den Tiegel 30 bewegen und das Wandeln der elektrischen Ausgabegröße des Temperaturfühlers 12 für die Steuereinheit (SPS).

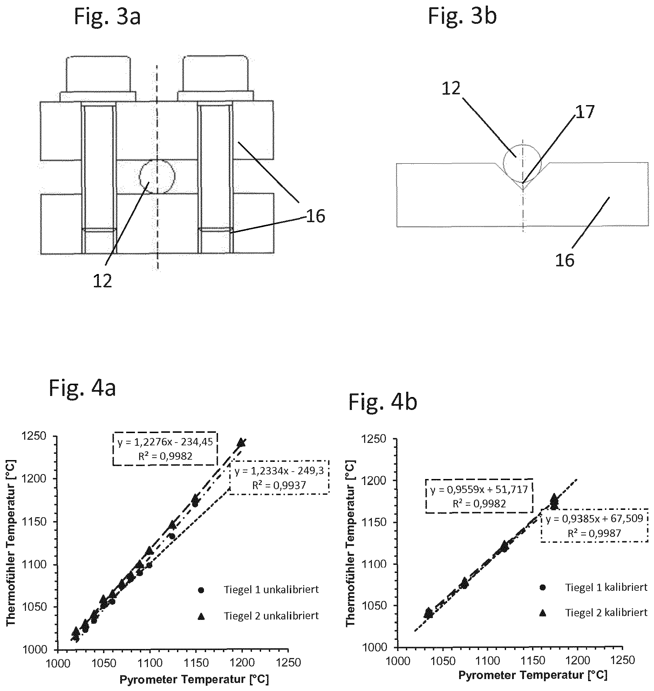

Der Temperaturfühler 12 muss zum Tiegel 30 definiert ausgerichtet werden (x- und y-Richtung), damit der Temperaturfühler 12 zum Tiegel bewegt werden kann. Eine Voraussetzung ist dabei, dass der Tiegel definiert in eine vordefinierte Tiegelhalterung eingesetzt wird, die bei einer Aufschlusseinrichtung bspw. durch eine Dreipunkthalterung für den Aufschlussvorgang ausgebildet wird. Erst durch die fest definierte Position des Tiegels kann der Temperaturfühler 12 dementsprechend ausgerichtet werden. Vorliegend wird für das Ausrichten des Temperaturfühlers 12 zum Tiegel der Temperaturfühler 12 am Mantel in eine ausgefräste Kerbe der Klemmbacke 16 gelegt. Durch die insbesondere dreiecksförmige Kerbe zentriert bzw. positioniert sich der Temperaturfühler 12 einfach (siehe Figur 2). Auch in z- Richtung, also in einer Richtung nach oben und unten bezogen auf den Tiegel in Figur 2 muss der Temperaturfühler 12 ausgerichtet werden. Die Ausrichtung des Temperaturfühlers 12 in der z-Achse wird beispielsweise mit Hilfe eines Anschlages realisiert. Ein Anschlag ist die einfachste Lösung für eine definierte Ausrichtung in der z-Achse, da alle Tiegel dieselbe Höhe haben. In Figur 2 wird die z-Richtung des gebogenen Temperaturfühlers 12 einerseits durch die Biegung definiert und andererseits anhand des Anschlages 18 der die Bewegung des Temperaturfühlers 12 begrenzt. Die Fixierung des Thermofühlers ist wichtig, damit sich die definierte Position des Thermofühlers nach dem Ausrichten nicht mehr verändert. Zudem muss darauf geachtet werden, dass keine zu hohen punktuellen Kräfte auf dem Thermofühler wirken. So wird bevorzugt nicht der Stecker des Temperaturfühlers 12 fixiert, sondern der Mantel. Der Mantel wird dabei mit Hilfe der Klemmbacken 16 befestigt, die vorzugsweise über zwei Schraubenverbindungen verbunden sind (siehe Fig. 3a).

Durch das Bewegen des ausgerichteten und fixierten Temperaturfühlers 12 wird ermöglicht, dass die Temperatur in der Mitte des Tiegels in der Schmelze gemessen wird. Der Temperaturfühler 12 wird dabei vorzugsweise um 90° gebogen damit eine Rotation möglich ist. Der innere Biegeradius beträgt dabei bevorzugt 10 mm. Das Biegen des Temperaturfühlers 12 ermöglicht die Rotation in dem geringen Bauraums des Aufschlussgeräts. Im ungebogenen Zustand lässt der geringe Bauraum keine Rotationsbewegung zu. Der Temperaturfühler 12 kann dann beispielsweise über eine Zugbewegung mittels der Betätigungsstange 20 aus bzw. in den Tiegel bewegt werden, so dass eine Rotation an der Drehachse 13 entsteht.

Das Umwandeln des elektrischen Signals wird bevorzugt mit Hilfe eines Transmitters realisiert. Dies ist die optimale Lösung, da sie einfach umzusetzen ist und eine hohe Prozesssicherheit gewährleistet. Zudem verfügt der Transmitter über eine Funktion, die detektiert, wenn das ausgegebene Signal des Temperaturfühlers 12 aufgrund von Verschleißerscheinungen nicht der programmierten Skalierung entspricht.

Für das Ausrichten des Temperaturfühlers 12 kann ein Deckel vorgesehen sein. Der Deckel ist bevorzugt aus Kunststoff Polyethylen verwendet. Der Deckel weist ein Loch auf, welches sich genau in der Mitte befinden sollte und durch das eine Relativposition des Temperaturfühlers 12 zum Tiegel 30 reproduzierbar ist. Der Temperaturfühler 12 Durch das Loch wird der gebogene Thermofühler gesteckt und anschließend fixiert. Das Ausrichten des Thermofühlers findet normalerweise vor einer Kalibrierung statt, da der Kunststoff nicht für die hohen Temperaturen von bis zu 1300 °C ausgelegt ist.

Da die Kalibrierungsvorrichtung ggf. nur bei Bedarf in dem Aufschlussgerät platziert werden soll, wird diese auf einer Fußplatte bzw. Standplattform 24 befestigt. Zur Überwindung der Höhendifferenz zwischen Fußplatte und der Temperaturfühleraufnahme können zwei Säulen 22 mit einer definierten Höhe auf die Fußplatte befestigt werden, bspw. durch Schrauben oder Schweißen.

Die Tiegelkalibration läuft im Einzelnen wie folgt ab: Über das Bedienpanel des Aufschlussgerätes oder ein übergeordnetes Steuerungssystem wird die Kalibration eines neuen Tiegels 30 angemeldet. Der Tiegel 30 wird entweder vom Bediener (manuelles Aufschlusssystem) oder von der in einer vollautomatischen Maschine enthaltenen Dosiervorrichtung mit dem gewünschten Inhalt gefüllt. Der Tiegel 30 wird in einen Induktionsofen in den Tiegelstandort eingesetzt, welcher mit der Messvorrichtung 10 ausgestattet ist. Diese ermöglicht, wie oben bereits ausgeführt, dass der Temperaturfühler 12 immer an der gleichen Position und gleichen Tiefe in der Schmelze des Tiegels 30 eingeführt wird. Der nachfolgende Vorgang mit Ansteuerung unterschiedlicher Temperaturstufen erfolgt durch die in der Maschine integrierte SPS. Wenn der Inhalt des Tiegels 30 geschmolzen ist, wird der Bediener aufgefordert, den Tauchfühler in die Schmelze einzutauchen. Dies erfolgt von außerhalb der Maschine, ohne den Bediener zu gefährden. In vollautomatischen Aufschlusssystemen taucht der Tauchfühler selbstständig in die Schmelze. Nachfolgend werden in aufsteigender Reihenfolge unterschiedliche Temperaturstufen in einem gewünschten Temperaturbereich angefahren. Bei Lithiumtetraborat (LiT) liegt dieser Bereich zwischen etwa 1000°C und 1200°C und es gibt etwa 4 Ansteuertemperaturen. Dabei erfolgt die Ansteuerung der vier Temperaturstufen entweder mit Hilfe der (noch unkalibrierten) Pyrometertemperatur oder durch die Leistungsausgabe des Hochfrequenzgenerators. Nach einem festgelegten zeitlichen Protokoll werden die Pyrometer- und Schmelzentemperatur für die jeweilige Temperaturstufe aufgenommen und in einem entsprechenden Datenbaustein der SPS abgespeichert.

Aus den aufgenommenen Werten (siehe Figur 4a für die Kalibration von zwei unterschiedlichen Tiegeln) kann dann die korrigierte Pyrometertemperatur berechnet werden. In Figur 4a ist der Zusammenhang zwischen "echter" Schmelztemperatur (gemessen durch Thermofühler) und nicht korrigierter Pyrometertemperatur bei Platin-/ Gold-Tiegel ohne vorherige Kalibration dargestellt.

Sowohl die gemessene Thermofühler- Temperatur als auch die Pyrometer- Temperatur zeigen einen linearen Verlauf. Daraus resultiert, dass der Zusammenhang zwischen beiden Temperaturen mit einer Geradengleichung (siehe Formel 1) beschrieben werden kann.

**Formel 1:**     Geradengleichung

$$y = m \cdot x + b$$

mit: y = Thermofühler-Temperatur
x = Pyrometer-Temperatur

Es werden die Steigung m und der y-Achsenabschnitt b bestimmt werden. Für die Berechnung der Parameter werden folgende Formeln verwendet:

**Formel 2:**     Mittelwertberechnung der Pyrometer-Temperatur

$$\bar{x} = \frac{1}{n}$$

**Formel 3:**     Mittelwertberechnung der Thermofühler-Temperatur

$$\bar{y} = \frac{1}{n}\sum_{i=1}^{n} y_i$$

Für die Berechnung der Mittelwerte $\bar{x}$ und $\bar{y}$ gilt hier n=4. Damit die Gleichung der Geraden möglichst repräsentativ ist, wird Formel 4 für die Berechnung der Steigung der Geraden herangezogen. Die Gleichung für m berücksichtigt alle aufgenommenen Werte.

**Formel 4:** Berechnung der Geradensteigung

$$m = \frac{\sum_{i=1}^{n}(x_i - \bar{x})(y_i - \bar{y})}{\sum_{i=1}^{n}(x_i - \bar{x})^2}$$

Ergebnis dieser Formel ist die Steigung m der Geraden für die Wertetabelle. Der y-Achsenabschnitt b wird wie folgt errechnet (Formel 5):

**Formel 5:** Berechnung des y Achsenabschnittes

$$b = m \cdot \bar{x} + \bar{y}$$

Ergebnis der Berechnungen ist eine Gleichung, die das Verhalten der Thermofühler-Temperatur in Abhängigkeit der Pyrometer-Temperatur beschreibt. Für die Kalibrierung wird die Gleichung nach x umgestellt (siehe Formel 6).

**Formel 6:** Geradengleichung nach x umgestellt

$$x = \frac{y - b}{m}$$

Mit der dargestellten Formel lässt sich für den kalibrierten Tiegel für jede beliebige Zieltemperatur im gewünschten Temperaturbereich eine dazugehörige "korrigierte Pyrometer-Temperatur" berechnen (im obigen Beispiel mit LiT ein Bereich von 1050-1125 °C). Dabei wird der Temperaturabhängigkeit des Emissionsgrades mit Hilfe der korrigierten Pyrometer-Temperatur entgegengewirkt. Zudem wird jedem Platintiegel nach Ablauf der Kalibration eine individuelle Geradengleichung zugeordnet, damit bei der Verwendung von verschiedenen Tiegeln keine Anpassung des Emissionsgrades notwendig ist.

Nach der Ermittlung der Geradengleichung werden die korrigierten Pyrometer-Temperaturen für die Zieltemperaturen errechnet (Im LiT Beispiel bei 1050, 1075, 1100 und 1125 °C). Für die Berechnung der korrigierten Pyrometer-Temperatur wird der jeweilige Zieltemperaturwert (1050, 1075, 1100 und 1125 °C) in die Gleichung (siehe Formel 6) für y eingesetzt. Nach der Berechnung der korrigierten Pyrometer- Temperaturen stimmen die Pyrometertemperaturen mit den Zieltemperaturen in der Schmelze überein (siehe Figur 4b, bei der die "echte" Schmelztemperatur (gemessen durch Thermofühler) und die korrigierter Pyrometer-Temperatur zeigen eine hohe Übereinstimmung zeigen.

Wenn die Steuereinheit des Pyrometers auch einen Eingang besitzt, kann auch der Emissionsgrad $\varepsilon$ kalibriert werden. Die Gesamtstrahlungsleistung P für einen realen, grauen Körper berechnet sich durch:

$$P = \varepsilon \cdot \sigma \cdot A \cdot T^4$$

Wobei die Stefan-Boltzmann-Konstante $\sigma = 5{,}6704 \cdot 10^{-8}$ W m$^{-2}$ K$^{-4}$, A die Fläche [m$^2$] und T [K] die Temperatur ist. Ein grauer Körper emittiert daher eine um den Faktor $\varepsilon$ geringere Energie als es ein schwarzer Körper würde, für welchen der Emissionsgrad $\varepsilon$ 1 beträgt.

Bei der Kalibrierung werden dann, analog zu der Kalibration nach Pyrometertemperatur oder Energiemenge, vorzugsweise 4 Temperaturstufen angesteuert. Anschließend wird über die SPS der Emissionsgrad $\varepsilon$ für jede Temperaturstufe angepasst, sodass die Thermofühlertemperatur der ausgegebenen Pyrometertemperatur entspricht. Anschließend wird der Emissionsgrad $\varepsilon$ in Verbindung mit Pyrometer- und Thermofühlertempertur gesetzt und eine Kalibrationsgerade berechnet.

Nach der initialen Kalibration des Tiegels erfolgt in regelmäßigen Intervallen, die nach der Anzahl der aufgeführten Aufschlüsse, Heizleistung oder anderen Parametern eingeteilt werden können, eine sogenannte Rekalibration. Die Rekalibration dient dazu, alterungsbedingte Änderungen des Emissionsgrades an der Außenseite des Tiegels zu kompensieren. Für die Rekalibration können zwei alternative Vorgehensweisen angewendet werden. Die erste Möglichkeit verwendet zur Rekalibration sowohl die Schmelztemperatur als auch die Pyrometer- Temperatur. Dann ist der gesamte Vorgang identisch zu vorhergehend aufgeführtem Verfahren.

Bei der zweiten Möglichkeit werden die vier Temperaturstufen nicht mit Hilfe der Pyrometertemperatur angefahren, sondern mit Hilfe der Hochfrequenzgeneratorleistung. Da genau bekannt ist, welche Temperaturen durch die entsprechende Leistung in der Schmelze des Tiegels verursacht werden, ist es dann ausreichend, lediglich die Pyrometer- Temperatur zu erfassen. Die Abweichung der gemessenen Pyrometer- Temperatur von der zu erwartenden Temperatur kann wiederum durch einen Korrekturfaktor ausgeglichen werden.

Bezugszeichenliste

Temperaturmessvorrichtung 10
Stecker 11
Temperaturfühler 12
Achse 13
Lagerplatte 14
Lagerelemente 15
Klemmbacke 16
Lagernut 17
Anschlag 18
Zwischenlagerung 19
Betätigungsstange 20
Beinen 22
Schild 23
Standplattform 24
Schlitz 25
Tiegel 30
Tiegelboden 31
Tiegelwand 32

**Patentansprüche**

1. Aufschlussvorrichtung, umfassend:

   eine Hitzestrahlungserfassungseinrichtung;
   eine Temperaturmessvorrichtung (10) zum Kalibrieren der Schmelztemperaturerfassung, wobei die Temperaturmessvorrichtung (10) umfasst:

   einen bewegbaren und zumindest teilweise stangenförmigen Temperaturfühler (12), der in einen Tiegel (30) einsteckbar ist;
   eine Bewegungseinrichtung zum Bewegen des Temperaturfühlers (12) zwischen einer Initialposition und einer Messposition;
   und eine Lagerplatte (14), auf der der stangenförmige Temperaturfühler (12) und die Bewegungseinrichtung gelagert sind und die in einer Aufschlussvorrichtung montierbar ist;
   wobei die Aufschlussvorrichtung weiter umfasst:

   einen Tiegelstellplatz, der in Bezug auf die Temperaturmessvorrichtung (10) vordefiniert ist;
   eine Induktionseinheit, mit der der Tiegelinhalt aufheizbar ist;
   eine Steuereinheit, die den Abgleich zwischen der durch die Temperaturmessvorrichtung (10) gemessenen Schmelztemperatur und der durch die Hitzestrahlungserfassungseinrichtung erfassten Strahlung durchführt.

2. Vorrichtung nach Anspruch 1, bei der der Temperaturfühler (12) mittels zweier Klemmbacken (16) fixiert ist.

3. Vorrichtung nach Anspruch 2, bei der der Temperaturfühler (12) in einer Nut (17) von zumindest einer Klemmbacke (16) angeordnet ist.

4. Vorrichtung nach einem der vorhergehenden Ansprüche, bei der der Temperaturfühler (12) gebogen ausgebildet ist.

5. Vorrichtung nach einem der vorhergehenden Ansprüche, ferner umfassend einen Hitzeschild (23), der eine Aussparung aufweist, durch die der Temperaturfühler hindurch bewegbar ist.

**6.** Vorrichtung nach einem der vorhergehenden Ansprüche, wobei der Temperaturfühler (12) durch die Bewegungseinrichtung rotatorisch gelagert ist.

**7.** Vorrichtung nach einem der vorhergehenden Ansprüche, ferner umfassend zumindest einen Anschlag (18) zum Begrenzen der Bewegung der Bewegungseinrichtung.

**8.** Verfahren zum Kalibrieren einer Temperaturmesseinrichtung (10) einer Aufschlussvorrichtung. umfassend die folgenden Schritte:

    - Bereitstellen eines Schmelztiegels (30) an einer vorbestimmten Stelle:
    - induktives Aufheizen eines Schmelztiegels (30) mit Inhalt, so dass der Inhalt sich erwärmt und schmilzt;
    - Anfahren eines Temperaturfühlers (12) an eine Messposition in der Schmelze und exaktes Messen der Schmelztemperatur mit dem Temperaturfühler (12);
    - Erfassen der Hitzestrahlung mit einer Hitzestrahlungserfassungseinrichtung; und
    - In Beziehung Setzen der erfassten Hitzestrahlung des Tiegels (30) zur gemessener Schmelztemperatur, so dass aus der Hitzestrahlung auf die Schmelztemperatur zurückgeschlossen werden kann.

**9.** Verfahren nach Anspruch 8, bei dem die Schritte des exakten Messens der Schmelztemperatur mit dem Temperaturfühler (12) und das Erfassen der Hitzestrahlung mit der Hitzestrahlungserfassungseinrichtung bei unterschiedlichen Temperaturen wiederholt werden.

**10.** Verfahren nach einem der Ansprüche 8 oder 9, bei dem das Verfahren zuerst mit einem unbenutzten Schmelztiegel (30) durchgeführt wird und dann in einem bestimmten Zeitabstand wiederholt wird, um die alterungsbedingte Änderung der Hitzestrahlungseigenschaften des Tiegels (30) zu erfassen.

**Claims**

**1.** Digestion device, comprising:

    a heat beam detection device;
    a temperature measuring device (10) for calibrating the melting temperature detection, the temperature measuring device (10) comprising:

        a movable and at least partially rod shaped temperature sensor (12), which is insertable into a crucible (30);
        a drive mechanism for moving the temperature sensor (12) between an initial position and a measurement position; and
        a base plate (14), the rod shaped temperature sensor (12) and the drive mechanism being based on the base plate (14), and the base plate (14) being mountable in a digestion device;
        wherein the digestion device further comprises
        a crucible space which is predefined in relation to the temperature measuring device (10);
        an induction unit, with which the contents of the crucible can be heated up;
        a control unit, which runs the balancing of the melting temperature measured by the temperature measuring device (10) and the beam detected by the heat beam detection device.

**2.** Device according to claim 1, wherein the temperature sensor (12) is fixed by means of two clamping jaws (16).

**3.** Device according to claim 2, wherein the temperature sensor (12) is positioned in a groove (17) of at least one clamping jaw (16).

**4.** Device according to any preceding claim, wherein the temperature sensor (12) is configured in a curved manner.

**5.** Device according to any preceding claim, further comprising a heat shield (23), which comprises a recess, wherein the temperature sensor is movable through the recess.

**6.** Device according to any preceding claim, wherein the temperature sensor (12) is rotationally mounted by means of the drive mechanism.

7.  Device according to any preceding claim, further comprising at least one stop (18) for restricting the movement of the drive mechanism.

8.  Procedure for calibrating a temperature measuring device (10) of a digestion device, comprising the following steps:

    - providing a crucible at a predefined position;
    - inductive heating of a crucible (30) with contents so that the content is heated up and melted;
    - moving a temperature sensor (12) into a measurement position in the melt and exact measurement of the melting temperature with the temperature sensor (12);
    - detecting the heat beam with a heat beam detection device; and
    - correlating the detected heat beam of the crucible (30) with the measured melting temperature, so that with the heat beam the melting temperature can be concluded.

9.  Procedure according to claim 8, wherein the steps of exact measurement of the melting temperature with the temperature sensor (12) and detecting the heat beam with a heat beam detection device are repeated at different temperatures.

10. Procedure according to claim 8 or 9, wherein the procedure is initially executed with an unused crucible (30) and then repeated at a certain time interval to detect the age-related change of the heat beam properties of the crucible (30).

**Revendications**

1.  Dispositif de dissolution, comprenant :

    un appareil de détection du rayonnement thermique ;
    un dispositif de mesure de la température (10) pour calibrer la détection de la température de fusion, le dispositif de mesure de la température (10) comprenant

    une sonde de température (12) mobile et au moins partiellement en forme de tige, qui peut être insérée dans un creuset (30) ;
    un appareil de déplacement pour déplacer la sonde de température (12) entre une position initiale et une position de mesure ;
    et une plaque de support (14) sur laquelle la sonde de température en forme de tige (12) et l'appareil de déplacement sont montés et qui peut être montée dans un dispositif de dissolution ;
    le dispositif de dissolution comprenant en outre :

    un espace de stationnement du creuset qui est prédéfini par rapport au dispositif de mesure de la température (10) ;
    une unité d'induction permettant de chauffer le contenu du creuset ;
    une unité de commande qui effectue la compensation entre la température de fusion mesurée par le dispositif de mesure de la température (10) et le rayonnement détecté par l'appareil de détection de rayonnement thermique.

2.  Dispositif selon la revendication 1, dans lequel la sonde de température (12) est fixée au moyen de deux mâchoires de serrage (16).

3.  Dispositif selon la revendication 2, dans lequel la sonde de température (12) est disposé dans une rainure (17) d'au moins une mâchoire de serrage (16).

4.  Dispositif selon l'une quelconque des revendications précédentes, dans lequel la sonde de température (12) est courbée.

5.  Dispositif selon l'une quelconque des revendications précédentes, comprenant en outre un bouclier thermique (23) ayant un évidement à travers lequel la sonde de température peut être déplacée.

6.  Dispositif selon l'une des revendications précédentes, dans lequel la sonde de température (12) est montée en rotation par l'appareil de déplacement.

**7.** Dispositif selon l'une quelconque des revendications précédentes, comprenant en outre au moins une butée (18) pour limiter le mouvement de l'appareil de déplacement.

**8.** Procédé de calibration d'un appareil de mesure de la température (10) d'un dispositif de dissolution, comprenant les étapes suivantes :

- fournir un creuset de fusion (30) à un emplacement prédéterminé ;
- chauffer par induction un creuset de fusion (30) avec un contenu tel que le contenu se réchauffe et fond ;
- approcher une sonde de la température (12) jusqu'à une position de mesure dans la masse fondue et mesurer précisément la température de fusion avec la sonde de température (12) ;
- détecter le rayonnement thermique à l'aide d'un appareil de détection du rayonnement thermique, et
- mettre en relation le rayonnement thermique détecté du creuset (30) et la température de fusion mesurée, de sorte que le rayonnement thermique puisse être ramené à la température de fusion.

**9.** Procédé selon la revendication 8, dans lequel les étapes consistant à mesurer avec précision la température de fusion avec la sonde de température (12) et à détecter le rayonnement thermique avec l'appareil de détection de rayonnement thermique sont répétées à des températures différentes.

**10.** Procédé selon l'une des revendications 8 ou 9, dans lequel le procédé est d'abord mis en œuvre avec un creuset de fusion (30) inutilisé, puis répété à un intervalle de temps prédéterminé pour détecter le changement lié au vieillissement des caractéristiques de rayonnement thermique du creuset (30).

Fig. 1a

Fig. 1b

Fig. 2

## Fig. 3a

## Fig. 3b

## Fig. 4a

## Fig. 4b

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1236982 A1